Europäisches Patentamt

European Patent Office

Office européen des brevets

(19)

(11) Publication number : **0 487 469 A1**

## (12) EUROPEAN PATENT APPLICATION

(21) Application number : **91830504.6**

(22) Date of filing : **18.11.91**

(51) Int. Cl.⁵ : **A61F 5/56**

(30) Priority : **23.11.90 IT 1944690 U**

(43) Date of publication of application :
**27.05.92 Bulletin 92/22**

(84) Designated Contracting States :
**AT BE CH DE DK ES FR GB GR LI LU NL SE**

(71) Applicant : **Cecconi, Sandro**
**Via Papa Giovanni XXIII, 12**
**I-63022 Falerone (AP) (IT)**

(72) Inventor : **Cecconi, Sandro**
**Via Papa Giovanni XXIII, 12**
**I-63022 Falerone (AP) (IT)**

(74) Representative : **Baldi, Claudio**
**Piazza Ghislieri, 3**
**I-60035 Jesi (Ancona) (IT)**

(54) **A device for keeping dental arches in an open and stable position during sleep.**

(57)   This invention concerns a device for keeping dental arches in an open and stable position during sleep. The device in question consists of a middle vertical bridge joining two overlying horizontal anatomically curved wings which couple the same to the upper and lower jaw arch.

FIG. 1

EP 0 487 469 A1

This invention concerns a device for keeping dental arches in an open and stable position during sleep, ideal for those with functional-anatomical problems of the respiratory naso-oral-pharyngeal air flow.

The device in question is an absolute novelty since there are currently no known devices which perform the above function.

The device in question acts symmetrically on the two dental arches, so that the same are positioned and kept on planes specified by the medical specialist.

The device in question, moreover, permits adjusting the relationship of the dental arches and the palate and oral floor, thereby ensuring major and freer air flow, above all in the case of functional problems due to old age or acquired anatomical problems such as obesity.

The device in question consists of an object shaped like an "H" overturned by 90 °, so as to have two overlying horizontal wings joined by a middle vertical bridge.

Each of the horizontal wings is curved so that it can be fitted anatomically to the dental arch.

The above vertical bridge acts as a spacer between the bottom wing coupled with the lower jaw arch and the upper wing coupled with the upper jaw arch, whereby the two dental arches are kept open at the distance required, which obviously corresponds to the height of said bridge.

This device is constructed by means of a supporting core made of a rigid material, such as steel, coated with a soft material like rubber or plastic.

This device also can be moulded in a single material, whose characteristics are suitable for the design purpose.

For major clarity, the description of the invention continues with reference to the enclosed drawings, which are intended for illustrative purposes and not in a limiting sense, whereby:

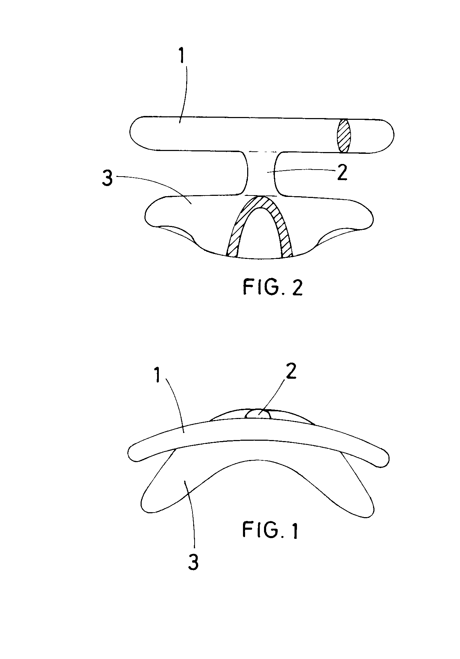

– fig. 1 is a perspective view and top view of the device, according to the invention;

– fig. 2 is a view of the device in question inside the mouth on a horizontal plane; this drawing illustrates the relevant cross-section above each of the two wings.

With reference to the above drawings, the device in question consists of an object having a curved upper horizontal wing (1) which fits anatomically to the shape of the upper jaw arch, against which it rests.

Said wing (1) is very fine in that it fits outside the teeth of the upper arch and inside the top lip.

From the upper wing (1) there is a shaped middle bridge (2), whose base joins with the curved bottom horizontal wing (3) which anatomically fits the shape of the lower jaw arch.

Said lower wing (3) has an upside down "U" shaped cross-section in that it is hollowed at the bottom and internally so as to house the teeth of the lower jaw arch, which is in this way wedged and functionally modulated by means of the connection bridge (2) on the upper jaw arch. The thickness and linear extension of the top wing (1) may change from time to time according to the tolerance of the subject, while the size and shape of the bottom wing (3) must always be suitable for housing the teeth of the lower jaw arch.

In a different embodiment of the invention, the middle bridge (2) may also have additional connection bridges placed side by side between the two wings (1 and 3).

## Claims

**1)** A device for keeping dental arches in an open and stable position during sleep, consisting of an upper horizontal wing (1) connected to a bottom horizontal wing (3) by means of a vertical middle bridge (2); the upper wing (1) has a curved anatomical shape permitting the same to couple with the upper jaw arch, and fitting outside the teeth and inside the upper lip; the bottom wing (3) has an anatomical shape and a hollow upside down "U" shape so as to house the teeth of the lower jaw arch.

**2)** A device for keeping dental arches in an open and stable position during sleep, according to claim 1), characterized in that the wings (1 and 3) are joined by a number of vertical bridges (2).

EP 0 487 469 A1

FIG. 2

FIG. 1

| | European Patent Office | EUROPEAN SEARCH REPORT | Application Number |
|---|---|---|---|

EP    91 83 0504

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-0 312 368 (HAYS & MEADE INC.) <br> * abstract; figures * <br> --- | 1,2 | A61F5/56 |
| A | GB-A-1 569 129 (RATA) <br> * page 1, line 26 - page 2, line 20; figures * <br> --- | 1,2 | |
| A | US-A-4 715 368 (GEORGE) <br> * abstract; figures * <br> --- | 1 | |
| A | US-A-4 901 737 (TOONE) <br><br> ----- | | |
| | | | TECHNICAL FIELDS SEARCHED (Int. Cl.5 ) <br><br> A61F <br> A61C |

The present search report has been drawn up for all claims

| Place of search | Date of completion of the search | Examiner |
|---|---|---|
| THE HAGUE | 28 FEBRUARY 1992 | SANCHEZ Y SANCHEZ J. |

EPO FORM 1503 03.82 (P0401)